(19) Europäisches Patentamt
European Patent Office
Office européen des brevets

(11) Veröffentlichungsnummer: **0 129 747**
**A2**

(12) # EUROPÄISCHE PATENTANMELDUNG

(21) Anmeldenummer: **84106419.9**

(22) Anmeldetag: **05.06.84**

(51) Int. Cl.⁴: **C 07 D 513/10**
C 07 D 277/60, A 61 K 31/425
//C07D311/22, C07D409/04,
(C07D513/10, 311/00, 277/00)

(30) Priorität: **23.06.83 CH 3432/83**
**13.04.84 CH 1860/84**

(43) Veröffentlichungstag der Anmeldung:
**02.01.85 Patentblatt 85/1**

(84) Benannte Vertragsstaaten:
**AT BE CH DE FR GB IT LI LU NL SE**

(71) Anmelder: **F. HOFFMANN-LA ROCHE & CO.**
**Aktiengesellschaft**

**CH-4002 Basel(CH)**

(72) Erfinder: **Hasler, Heinz, Dr.**
**Liebrütistrasse 40**
**CH-4303 Kaiseraugst(CH)**

(72) Erfinder: **Schneider, Fernand, Dr.**
**Marignanostrasse 28**
**CH-4059 Basel(CH)**

(74) Vertreter: **Lederer, Franz, Dr. et al,**
**Patentanwälte Dr. Franz Lederer Dipl.-Ing. Reiner F.**
**Meyer-Roxlau Lucile-Grahn-Strasse 22**
**D-8000 München 80(DE)**

(54) **Thiazolidinderivate, deren Herstellung und Zwischenprodukte dafür sowie pharmazeutische Präparate.**

(57) Die neuen Verbindungen der allgemeinen Formel

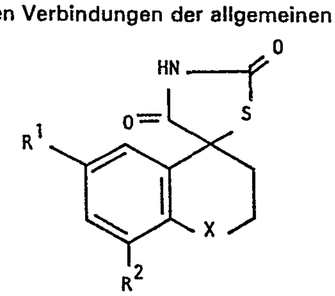

I

worin X Methylen, Sauerstoff oder Schwefel; $R^1$ Halogen; $R^2$ Wasserstoff, Nitro, Cyano, $OR^3$ oder $NHR^3$; oder beide Reste $R^1$ und $R^2$ gleichzeitig Wasserstoff oder Nitro; $R^3$ Wasserstoff, Alkyl, Acyl oder eine Gruppe $-CO-OR^4$ oder $-CO-NHR^4$; und $R^4$ Alkyl, Aryl oder Aralkyl bedeuten,

und deren Salze mit physiologisch verträglichen Kationen sind Aldosereduktasehemmer und können bei der Behandlung des Diabetes verwendet werden.

EP 0 129 747 A2

0129747

F.Hoffmann-La Roche & Co.Aktiengesellschaft,



RAN 4371/33

# Thiazolidinderivate, deren Herstellung und Zwischenprodukte dafür sowie pharmazeutische Präparate

Die Erfindung betrifft neue Thiazolidinderivate der allgemeinen Formel

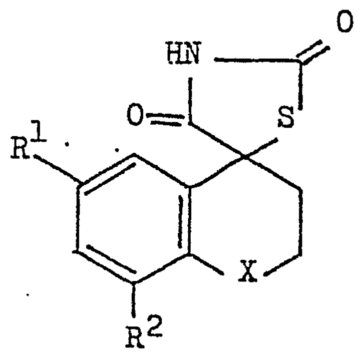

I

worin X Methylen, Sauerstoff oder Schwefel; $R^1$ Halogen; $R^2$ Wasserstoff, Nitro, Cyano, $OR^3$ oder $NHR^3$; oder beide Reste $R^1$ und $R^2$ gleichzeitig Wasserstoff oder Nitro; $R^3$ Wasserstoff, Alkyl, Acyl oder eine Gruppe $-CO-OR^4$ oder $-CO-NHR^4$; und $R^4$ Alkyl, Aryl oder Aralkyl bedeuten,

und deren Salze mit physiologisch verträglichen Kationen.

Die Erfindung betrifft weiterhin ein Verfahren zur Herstellung von Verbindungen der Formel I sowie Salzen davon, pharmazeutische Präparate auf der Basis solcher Verbindungen und diese Verbindungen zur Anwendung als Heilmittel, insbesondere bei der Behandlung von Stoffwechselerkrankungen.

Grn/ 3.5.84

Von den Halogensubstituenten sind Fluor und Chlor bevorzugt. Alkylreste können geradkettig oder verzweigt sein und enthalten vorzugsweise 1-12 Kohlenstoffatome. Beispiele solcher Reste sind Methyl, Aethyl, Propyl, Butyl, Pentyl, Hexyl, Decyl und Dodecyl. Acylreste können sich von Carbonsäuren, z.B. aliphatischen, aromatischen oder araliphatischen Carbonsäuren mit vorzugsweise 1-12 Kohlenstoffatomen wie Essigsäure, Propionsäure, Buttersäure, Capronsäure, Undecylsäure, Dodecylsäure, Acetessigsäure, Acetylglykolsäure, Benzoesäure, und substituierte Benzoesäuren wie p-Toluylsäure, p-Nitrobenzoesäure, p-Chlorbenzoesäure, p-Brombenzoesäure und m-Dinitrobenzoesäure; Naphthoesäure, Phenylessigsäure; oder aliphatischen oder aromatischen Sulfonsäuren wie Methansulfonsäure, Aethansulfonsäure, Benzolsulfonsäure oder p-Toluolsulfonsäure ableiten. Durch $R^4$ dargestellte Alkylreste enthalten vorzugsweise 1- 6 C-Atome wie Methyl, Aethyl, Propyl, Isopropyl, Butyl und Isobutyl. Beispiele von Arylresten sind Phenyl und substituierte Phenylreste, wie Halogenphenyl, Nitrophenyl, Alkoxycarbonyl-phenyl. Beispiele von Aralkylresten sind $C_{1-6}$-Alkylreste die durch einen der vorstehend erwähnten Arylreste substituiert sind, insbesondere Benzyl und Nitrobenzyl. Beispiele von physiologisch verträglichen Kationen sind Alkalimetall- und Erdalkalimetallionen wie $Na^+$, $K^+$, $Ca^{++}$ und $Mg^{++}$.

Von besonderem Interesse sind die Verbindungen der Formel I, in denen X Methylen, Sauerstoff oder Schwefel; $R^1$ Halogen; $R^2$ Wasserstoff, Nitro, Cyano oder $NHR^3$; und $R^3$ Wasserstoff, Alkyl oder Acyl bedeuten.

Bevorzugte Verbindungen der Formel I sind diejenigen, in denen X Sauerstoff ist; weiterhin diejenigen, in denen $R^1$ Halogen, insbesondere Fluor, ist. Von besonderem Interesse ist das 6-Fluor-8-acetamido-2,3-dihydrospiro-[4H-1-benzopyran-4,5'-thiazolidin]-2',4'-dion und das (±)-Methyl-6-fluor-2,3-dihydro-2',4'-dioxospiro[4H-1-benzopyran-4,5'-thiazolidin]-8-carbamat.

Die Verbindungen der Formel I und ihre Salze können als Racemate und in optisch aktiver Form vorliegen. Optisch aktive Verbindungen der Formel I können aus den Racematen in an sich bekannter Weise durch Racematspaltung, z.B. durch Chromatographie an einem optisch aktiven Träger oder durch Umsetzung mit optisch aktiven Basen wie Brucin, Trennung der diastereomeren Salze durch fraktionierte Kristallisation und Umwandlung der Salze in die freie Säure, d.h. die optisch aktive Verbindung der Formel I erhalten werden. Sie können auch dadurch erhalten werden, dass man als Ausgangsmaterial eine optisch aktive Verbindung der Formel II verwendet. Die optisch aktive Verbindung der Formel II kann ihrerseits durch Racematspaltung erhalten werden.

Die Verbindungen der Formel I können erfindungsgemäss dadurch hergestellt werden, dass man eine Verbindung der allgemeinen Formel

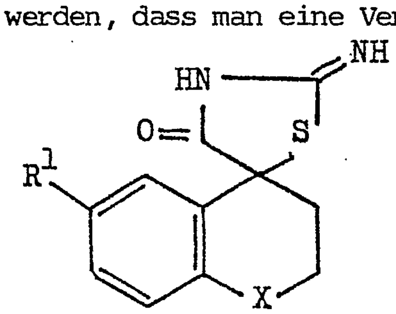

II

worin $R^1$ und X die oben angegebene Bedeutung haben, mit einer Säure behandelt, gewünschtenfalls das Reaktionsprodukt nitriert, gewünschtenfalls in einer so erhaltenen Verbindung der Formel I, in der $R^1$ Halogen ist, die Nitrogruppe zur Aminogruppe reduziert und letztere gewünschtenfalls acyliert oder alkyliert oder durch die Cyano- oder Hydroxygruppe ersetzt und letztere gewünschtenfalls alkyliert oder acyliert; und gegebenenfalls das Reaktionsprodukt in ein Salz mit einem physiologisch verträglichen Kation überführt.

Die Hydrolyse der Iminogruppe in einer Verbindung der Formel II kann durch Behandlung mit starken Säuren, wie Mineralsäuren, z.B. wässrig-alkoholischer Salzsäure, vorzugsweise unter Erwärmen bis zur Rückflusstemperatur des Reaktionsgemischs, vorgenommen werden. Eine so erhaltene Verbindung der Formel I, in der $R^1$ Halogen oder Wasser-

stoff und $R^2$ Wasserstoff darstellen, kann zu einer Verbindung der Formel I, in der $R^2$ bzw. $R^1$ und $R^2$ eine Nitrogruppe darstellen, nitriert werden. Diese Nitrierung kann in für die Nitrierung aromatischer Verbindungen an sich bekannter Weise durchgeführt werden, z.B. durch Behandlung mit einem Nitrierungsmittel wie starker Salpetersäure bei Temperaturen von etwa -20°C bis etwa Raumtemperatur. Eine Verbindung der Formel I, in der $R^1$ Halogen und $R^2$ Nitro ist, kann in für die Reduktion aromatischer Nitroverbindungen an sich bekannter Weise reduziert werden. Geeignete Reduktionsmittel sind z.B. Wasserstoff in Gegenwart von Katalysatoren, insbesondere Edelmetallkatalysatoren wie $PtO_2$ oder Pd-Katalysatoren; oder nascierender Wasserstoff, z.B. Fe/HCl, Raney-Nickel; oder Ammoniumsulfid in wässrigem Alkohol.

Die Aminogruppe $R^2$ in einer so erhaltenen Verbindung der Formel I kann in an sich bekannter Weise durch Behandlung mit einem Alkylierungsmittel oder einem Acylierungsmittel alkyliert bzw. acyliert werden. Geeignete Alkylierungsmittel sind Alkylhalogenide wie Methyl- oder Aethyljodid in Gegenwart einer Base, wie Kaliumcarbonat oder Pyridin in einem inerten Lösungsmittel, z.B. Aethanol. Beispiele für Acylierungsmittel sind reaktionsfähige Säurederivate wie Säureanhydride oder -halogenide, aktivierte Ester, z.B. Nitrophenylester. Der Ersatz der Aminogruppe durch die Cyano- oder Hydroxygruppe kann in an sich bekannter Weise durch Diazotierung und Umsetzung des Diazoniumsalzes mit Kupfer(I)-cyanid bzw. zwecks Einführung der Hydroxygruppe mit Kupfersalzen wie Kupfer(II)-nitrat durch Kupfer(I)-Katalyse bewerkstelligt werden. Die Acylreste $-COOR^4$ und $CONHR^4$ können durch Umsetzung einer Verbindung der Formel I, in der $R^2$ eine Amino- oder Hydroxygruppe darstellt, mit einem Isocyanat der Formel $R^4NCO$ bzw. einem Chlorameisensäureester der Formel $R^4OCOCl$ eingeführt werden.

Die Verbindungen der Formel II sind neu und ebenfalls Gegenstand der Erfindung. Sie können wie im Formelschema I dargestellt hergestellt werden.

Formelschema I

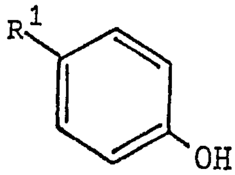

(1)

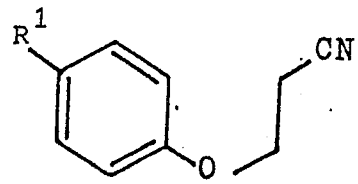

(2)

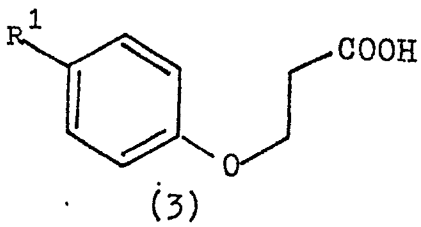

(3)

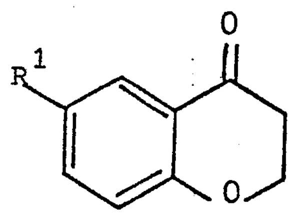

(4)

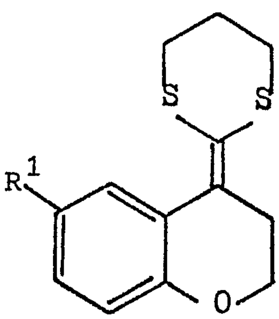

(5)

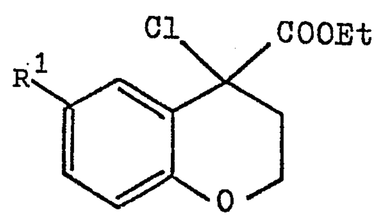

(6)

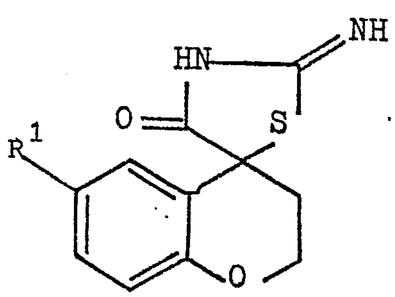

(7)

Eine Verbindung (1) kann durch Behandlung mit Acrylnitril in Gegenwart einer Base wie Natriummethylat unter Erwärmen auf Rückflusstemperatur in die Verbindung (2) übergeführt werden. Hydrolyse des Nitrils (2) durch Rückflusserhitzen mit starker wässriger Salzsäure liefert die Verbindung (3), aus der durch Behandlung mit heisser Polyphosphorsäure (4) erhalten wird. Der Aufbau der Thiazolidin-Gruppe geschieht schrittweise über eine Peterson-Olefinierung mit 2-Lithio-2-trimethylsilyl-1,3-dithian zu (5). Oxidation mit N-Chlorsuccinimid in Gegenwart eines Alkohols, vorzugsweise Aethanol, liefert den nicht sehr stabilen α-Chlor-Ester (6). Dieser wird längere Zeit in Gegenwart von Thioharnstoff in einem inerten Lösungsmittel wie Aceton gekocht, wobei das Chlor-Atom durch die Thioharnstoffgruppe ersetzt wird und spontane Cyclisierung zur spiro-Imino-Verbindung (7) erfolgt. Das Produkt fällt dabei als Hydrochlorid an; die freie Base wird nach Chromatographie an Kieselgel isoliert. In analoger Weise können die Verbindungen der Formel II mit X=S ausgehend von den Phenolen (1) entsprechenden Thiophenolen und die Verbindungen II mit X= Methylen ausgehend von den Chromanonen (4) entsprechenden Tetralonen hergestellt werden.

Die Verbindungen der Formel I und ihre Salze sind physiologisch, insbesondere als Aldosereduktase-Hemmer wirksam.

Bei der Prüfung auf Hemmwirkung bei der Aldosereduktase aus Kälber-Linsen (J. Biol. Chem. 240, 877 (1965)) wurden für die Verbindungen der Formel I mit $R^1$ = F und $R^2$ = H, $NO_2$ und $NHCOCH_3$ $IC_{50}$-Werte (Konzentrationen, die 50% Enzymhemmung bewirken) von 0,10 μM bzw. 0,089 und 0,008 μM gefunden.

Weiterhin wurde die Verbindung der Formel I mit $R^1$ = F und $R^2$ = H auf die Wirksamkeit an galactosämischen Ratten (30% Galactose-Diät) untersucht. Dabei wurde die Dulcit-Konzentration in der Augenlinse nach 5 Applikationen von

5 sowie von 20 mg/kg Körpergewicht per os über 2 Tage bestimmt. Ebenso wurden in einem weiteren Versuch 5 mal 20 mg/kg intraperitoneal verabreicht. In allen Versuchen wurde eine signifikante Verminderung der Dulcit-Konzentration in der Augenlinse gegenüber unbehandelten galactosämischen Tieren festgestellt. Die gleiche Verbindung wurde auch an mit Streptozotocin behandelte, diabetische Ratten appliziert (3 mal 20 mg/kg p.o., 4, 8 und 24 Stunden nach Streptozotocin-Verabreichung). 3 Stunden nach der letzten Dosis wurde die Sorbit-Konzentration in der Augenlinse und im Ischias-Nerv bestimmt. In beiden Organen war eine deutliche Verminderung der Sorbit-Konzentration gegenüber unbehandelten, diabetischen Tieren nachzuweisen.

Die Verbindungen der Formel I und ihre physiologisch verträglichen Salze können als Wirkstoffe in pharmazeutischen Präparaten, insbesondere zur Behandlung von Diabetes mellitus und zur Verhütung der dabei auftretenden Komplikationen wie Katarakt und Neuropathien verwendet werden. Die Verbindungen können oral oder parenteral in Dosen von 0,1-5 mg/kg Körpergewicht unter Berücksichtigung der Applikationsart und der Bedürfnisse des Patienten verabreicht werden. Die pharmazeutischen Präparate können z.B. in Form von Tabletten, Dragées, Kapseln, Infusionslösungen, Augentropfen oder -salben vorliegen, die neben dem Wirkstoff in solchen Präparaten übliche Hilfs- und Trägerstoffe enthalten und mit üblichen galenischen Techniken hergestellt werden können.

Die nachstehenden Beispiele erläutern die Erfindung weiter.

Beispiel 1

2,195 g (±)-6-Fluor-2,3-dihydro-2'-iminospiro[4H-1-benzopyran-4,5'-thiazolidin]-4'-on wurden in 12 ml Methanol und 12 ml 37%iger wässriger Salzsäure gelöst und über Nacht gekocht. Dabei schieden farblose Kristalle aus, die abgetrennt und mit Wasser gewaschen wurden. Nach Trocknen im Vakuum wurde (±)-6-Fluor-2,3-dihydrospiro[4H-1-benzopyran-4,5'-thiazolidin]-2',4'-dion vom Schmelzpunkt 147-149°C erhalten (95% d.Th.).

Das Ausgangsmaterial wurde wie folgt hergestellt:

Zu 112,1 g 4-Fluor-phenol wurden bei 60°C 5,4 g Natriummethylat portionsweise zugegeben. Das Gemisch wurde nach 10 Minuten mit 500 ml Acrylonitril versetzt, im Oelbad während 48 Stunden gekocht, danach auf 3 l Wasser gegossen und mit Chloroform extrahiert. Nach Trocknen der organischen Phase mit Natriumsulfat wurde im Vakuum vollständig eingeengt. Man erhielt 3-(4-Fluor-phenoxy)propionitril als viskoses Oel.

155 g 3-(4-Fluor-phenoxy)propionitril wurden in 5 l heisse 25%ige wässerige Salzsäure eingetragen und während 2 Stunden gekocht. Die Lösung wurde über Nacht stehen gelassen, wobei die 3-(4-Fluor-phenoxy)propionsäure in farblosen Nadeln, Schmelzpunkt 84-85°C, kristallisierte.

In 1,5 kg Polyphosphorsäure wurden bei 100°C unter kräftigem Rühren 135 g 3-(4-Fluor-phenoxy)propionsäure portionsweise eingetragen; dabei stieg die Temperatur auf 116°C. Nach 30 Minuten wurde das Reaktionsgemisch auf 6 l Eiswasser gegossen und mit 4 Portionen Chloroform extrahiert. Der Extrakt wurde mit Natriumsulfat getrocknet und vollständig eingeengt, der Rückstand wurde in Aethanol gelöst und mit 10 g Aktivkohle gekocht. Die filtrierte Lösung wurde heiss bis zur Sättigung konzentriert und dann stehen gelassen, wobei 81 g (6-Fluor-4-chromanon) vom

Schmelzpunkt 110-112°C kristallisierten. Eine Probe des braunroten Produktes wurde im Hochvakuum bei 100°C sublimiert und lieferte farblose Kristalle, Schmelzpunkt 113-114°C.

28,8 g 2-Trimethylsilyl-1,3-dithian wurden in 290 ml peroxydfreiem Tetrahydrofuran gelöst und bei -60°C tropfenweise mit 93 ml einer 1,6M Lösung von n-Butyllithium in Hexan versetzt. Das Reaktionsgemisch wurde innerhalb von 3 Stunden auf 0°C erwärmen gelassen und anschliessend wieder auf -60°C gekühlt. Eine Lösung von 24,87 g 6-Fluor-4-chromanon in 150 ml Tetrahydrofuran wurde anschliessend langsam zugetropft und das Gemisch unter Rühren über Nacht langsam von -60°C auf Raumtemperatur erwärmen gelassen. Anschliessend wurde auf 2,5 l Wasser gegossen und mit 4 Portionen Essigester extrahiert; die organischen Extrakte wurden mit gesättigter Kochsalzlösung gewaschen und mit Natriumsulfat getrocknet. Der eingeengte Extrakt wurde in einem Gemisch von Aether und Petroläther (tiefsiedend) 1:1 mit Aktivkohle entfärbt und aus demselben Gemisch in der Kälte zum Kristallisieren gebracht. Nach zwei Umkristallisationen wurden die vereinigten Mutterlaugen an einer Kieselgel 60-Säule chromatographiert (Hexan/Essigester 9:1). Die Produkt enthaltenden Fraktionen wurden konzentriert und umkristallisiert. Man erhielt 4-m-Dithian-2-yliden-6-fluor-2,3-dihydro-4H-1-benzopyran vom Schmelzpunkt 47-49°C.

Zu 26,7 g frisch kristallisiertem N-Chlorsuccinimid gelöst in 210 ml Acetonitril und 105 ml Aethanol, wurde bei Raumtemperatur eine Lösung von 10,73 g 4-m-Dithian-2-yliden-6-fluor-2,3-dihydro-4H-1-benzopyran in 107 ml Tetrahydrofuran langsam zugetropft. Das Reaktionsgemisch wurde 3 Stunden bei Raumtemperatur gerührt, anschliessend durch Zugabe von 100 ml Wasser hydrolysiert, mit 1 l Wasser verdünnt und mit 3 Portionen Essigester extrahiert. Die organischen Phasen wurden zweimal mit Wasser und einmal mit gesättigter Kochsalzlösung gewaschen, mit Natriumsulfat

getrocknet und im Vakuum zur Trockene eingeengt. Ueberschüssiges N-Chlorsuccinimid und entstandenes Succinimid wurden abgetrennt mittels Filtration durch eine Schicht Kieselgel 60 mit Hexan/Essigester 19:1. Der so erhaltene rohe α-Chlor-äthylester wurde in 85 ml Aceton gelöst, mit 2,48 g Thioharnstoff versetzt und während 4 Tagen gekocht. Die anfangs klare Lösung schied dabei farblose Kristalle aus. Diese wurden abgetrennt, mit Aceton gewaschen und aus Aethanol/Essigester umkristallisiert, wobei (±)-6-Fluor-2,3-dihydro-2'-iminospiro[4H-1-benzopyran-4,5'-thiazolidin]-4'-on-hydrochlorid vom Schmelzpunkt 215-217°C erhalten wurde.

Das Filtrat wurde eingeengt und der Rückstand an 500 g Kieselgel 60 chromatographiert (Chloroform/Methanol 9:1). Eine erste Fraktion ($R_f$ = 0,82) enthielt nicht umgesetztes Ausgangsmaterial zusammen mit dessen Zersetzungsprodukten, aus der Hauptfraktion ($R_f$ = 0,31) wurde durch Kristallisation aus Aethanol/Essigester die freie Base vom Schmelzpunkt 255-258°C isoliert. Eine Probe dieses Materials wurde bei 200-210°C/$10^{-2}$ mbar sublimiert und schmolz bei 261-263°C.

## Beispiel 2

In 2,5 ml rauchende Salpetersäure (96%, d 1.50), welche unter Rühren auf -20°C gekühlt war, wurden 500 mg (±)-6-Fluor-2,3-dihydrospiro[4H-1-benzopyran-4,5'-thiazolidin]-2',4'-dion so rasch eingetragen, dass die Temperatur -15°C nicht überstieg. Nach 3 Minuten Rühren bei -20°C wurde eine klare Lösung erhalten, die dann sofort auf 15 ml Eiswasser gegossen wurde. Nach kurzem Rühren wurde der entstandene Niederschlag abfiltriert, mit kaltem Wasser gewaschen und getrocknet. Man erhielt (±)-6-Fluor-8-nitro-2,3-dihydrospiro[4H-1-benzopyran-4,5'-thiazolidin]-2',4'-dion als gelbe Kristalle, welche aus Essigester/Hexan umkristallisiert bei 192-194°C schmolzen.

Beispiel 3

a) 755 mg (±)-6-Fluor-8-nitro-2,3-dihydrospiro[4H-1-benzopyran-4,5'-thiazolidin]-2',4'-dion wurden in Gegenwart von 50 mg Platinoxid in 50 ml Aethanol bei Raumtemperatur und Normaldruck hydriert. Nach 30 Minuten wurden weitere 100 mg Platinoxid zugegeben. Nach 2,5 Stunden unter Wasserstoff wurde nach Zugabe von etwas Chloroform vom Katalysator abfiltriert, eingeengt und mit Chloroform/Methanol 19:1 an Kieselgel 60 chromatographiert. Man erhielt als gelblichen Schaum (±)-6-Fluor-8-amino-2,3-dihydrospiro[4H-1-benzopyran-4,5'-thiazolidin]-2',4'-dion. Das IR-Spektrum zeigt Amin-Banden bei 3383, 3202 und 2766 $cm^{-1}$, im Massenspektrum erscheint das Molekular-Ion als Basisspeak (m/e = 268).

b) 100 mg (±)-6-Fluor-8-nitro-2,3-dihydrospiro[4H-1-benzopyran-4,5'-thiazolidin]-2',4'-dion wurden zusammen mit 57 mg Eisenpulver in 500 µl Aethanol und 250 µl Wasser aufgenommen. Nach Zugabe von 250 µl 0,16N Salzsäure wurde das Gemisch während 2 Stunden auf dem Oelbad bei 100°C gehalten. Anschliessend wurde abfiltriert, das Filtrat wurde mit 3 Portionen Essigester ausgeschüttelt, mit Natriumsulfat getrocknet und nach Konzentration an Kieselgel chromatographiert (Essigester/Aethanol 9:1). Das Produkt war mit dem in Abschnitt a) erhaltenen identisch.

c) 12 g (±)-6-Fluor-8-nitro-2,3-dihydrospiro[4H-1-benzopyran-4,5'-thiazolidin]-2',4'-dion wurden in 250 ml Aethanol aufgenommen und durch Zugabe von 25 ml 25%igem wässerigen Ammoniak in Lösung gebracht, Dazu wurde eine Lösung von 60 g Natriumsulfid und 13,37 g Ammoniumchlorid in 250 ml Wasser gegossen; anschliessend wurde das Reaktionsgemisch während 3 Stunden gekocht. Der Ansatz wurde auf 2 l kaltes Wasser gegossen und durch Zugabe von 25%iger Salzsäure angesäuert bis pH 5-5,5. Das Produkt wurde mit drei Portionen Essigester ausgeschüttelt, die organischen Phasen wurden mit ges. Kochsalzlösung gewaschen und mit Natriumsulfat getrocknet.

Nach Entfernen des Lösungsmittels wurde der Rückstand aus Chloroform umkristallisiert und lieferte beige Kristalle vom Smp. 179-181°C. Das Material entsprach in allen spektroskopischen Daten den in den Abschnitten a) und b) beschriebenen Produkten.

Beispiel 4

61 mg (±)-6-Fluor-8-amino-2,3-dihydrospiro[4H-1-benzopyran-4,5'-thiazolidin]-2',4'-dion wurden in einem Gemisch von 1,2 ml Eisessig und 1,2 ml Essigsäureanhydrid während 90 Minuten auf 50°C erwärmt und anschliessend in Vakuum zur Trockene eingeengt. Nach kurzem Aufkochen mit Aktivkohle in methanolischer Lösung wurde der Rückstand aus Methanol kristallisiert und lieferte (±)-6-Fluor-8-acetamido-2,3-dihydrospiro[4H-1-benzopyran-4,5'-thiazolidin]-2',4'-dion leicht bräunliches Material, Smp. 278-280°C.

Beispiel 5

In Analogie zu Beispiel 4 wurde aus (±)-6-Fluor-8-amino-2,3-dihydrospiro[4H-1-benzopyran-4,5'-thiazolidin]-2',4'-dion und Essigsäureameisensäureanhydrid in Ameisensäure das (±)-N-[6-Fluor-2,3-dihydro-2',4'-dioxospiro[4H-1-benzopyran-4,5'-thiazolidin]-8-yl]formamid, Smp. 144-146°C erhalten.

Beispiel 6

In Analogie zu Beispiel 1 wurde (±)-6-Chlor-2,3-dihydrospiro[4H-1-benzopyran-4,5'-thiazolidin]-2',4'-dion, Smp. 161-163°C aus (±)-6-Chlor-2,3-dihydro-2'-iminospiro[4H-1-benzopyran-4,5'-thiazolidin]-4'-on erhalten. Das Ausgangsmaterial wurde aus 6-Chlor-4-chromanon über 4-m-Dithian-2-yliden-6-chlor-2,3-dihydro-4H-1-benzopyran, Smp. 87-89°C, hergestellt.

## Beispiel 7

161 mg (±)-6-Fluor-8-amino-2,3-dihydrospiro[4H-1-benzopyran-4,5'-thiazolidin]-2',4'-dion wurden in 3,5 ml Pyridin gelöst und mit 135 mg Undecansäurechlorid versetzt. Nach 6 Stunden Rühren bei Raumtemperatur wurde die Hauptmenge des Pyridins im Vakuum entfernt, der Rückstand wurde mit 3 Portionen Essigester und 2 Portionen 1N Salzsäure extrahiert, die organischen Phasen wurden mit Wasser und mit gesättigter Kochsalzlösung neutral gewaschen, mit Natriumsulfat getrocknet und eingeengt. Der Rückstand wurde mit Chloroform/Methanol 19:1 an Kieselgel chromatographiert. Mit $R_f$ = 0,27 eluierten 182 mg Material in Form eines Harzes, das aus Methanol (±)-6-Fluor-8-undecylamido-2,3-dihydrospiro[4H-1-benzopyran-4,5'-thiazolidin]-2',4'-dion als farblose Kristalle vom Schmelzpunkt 69-71°C lieferte.

## Beispiel 8

In Analogie zu Beispiel 7 wurden hergestellt:

(±)-N-[6-Fluor-2,3-dihydro-2',4'-dioxospiro[4H-1-benzopyran-4,5'-thiazolidin]-8-yl]pivalamid, amorpher Feststoff, MS: m/e = 352: $M^+$, 61%; m/e = 57: 100%;

(±)-N-[6-Fluor-2,3-dihydro-2',4'-dioxospiro[4H-1-benzopyran-4,5'-thiazolidin]-8-yl]-3,3-dimethylbutyramid, amorpher Feststoff, MS: m/e = 366: $M^+$, 30%, m/e = 268: 100%; m/e = 57: 79%;

(±)-N-[6-Fluor-2,3-dihydro-2',4'-dioxospiro[4H-1-benzopyran-4,5'-thiazolidin]-8-yl]benzamid, Smp. 270-272°C.

(±)-p-Brom-N-2,3-dihydro-6-fluor-2',4'-dioxospiro[4H-1-benzopyran-4,5'-thiazolidin-8'-yl]benzamid, Smp. 297-298°C.

(±)-N-[6-Fluor-2,3-dihydro-2',4'-dioxospiro[4H-1-benzopyran-4,5'-thiazolidin]-8-yl]-p-nitrobenzamid, Smp. über 300°C, MS: m/e = 417: 47%; m/e = 150: 100%.

(±)-N-[6-Fluor-2,3-dihydro-2',4'-dioxospiro[4H-1-benzopyran-4,5'-thiazolidin]-8-yl]-3,5-dinitrobenzamid, Smp. 220-224°C.

(±)-[[6-Fluor-2,3-dihydro-2',4'-dioxospiro[4H-1-benzopyran-4,5'-thiazolidin]-8-yl]carbamoyl]methylacetat, Smp. 221-223°C.

## Beispiel 9

Zu einer Lösung von 536 mg (±)-6-Fluor-8-amino-2,3-dihydrospiro[4H-1-benzopyran-4,5'-thiazolidin]-2',4'-dion in 5,3ml Pyridin wurden 210 µl Chlorameisensäure-äthylester zugetropft. Das Reaktionsgemisch wurde über Nacht bei Raumtemperatur gerührt. Die Hauptmenge des Pyridins wurde im Vakuum entfernt und der Rückstand in Essigester aufgenommen und mit 0,2N-Salzsäure und gesättigter Kochsalzlösung gewaschen. Die vereinigten organischen Phasen wurden mit Natriumsulfat getrocknet und anschliessend eingeengt. Nach chromatographischer Reinigung an einer Säule mit Kieselgel (Elution mit Chloroform-Essigester 9:1) wurde das (±)-Aethyl-6-fluor-2,3-dihydro-2',4'-dioxospiro-[4H-1-benzopyran-4,5'-thiazolidin]-8-carbamat aus Essigester/n-Hexan umkristallisiert und lieferte farblose Kristalle vom Smp. 170-172°C.

## Beispiel 10

In Analogie zu Beispiel 9 wurden hergestellt:

(±)-Methyl-6-fluor-2,3-dihydro-2',4'-dioxospiro[4H-1-benzopyran-4,5'-thiazolidin]-8-carbamat, Smp. 128-130°C.

(±)-Butyl-6-fluor-2,3-dihydro-2',4'-dioxospiro[4H-1-benzopyran-4,5'-thiazolidin]-8-carbamat, Smp. 132-134°C.

(±)-Isobutyl-6-fluor-2,3-dihydro-2',4'-dioxospiro[4H-1-benzopyran-4,5'-thiazolidin]-8-carbamat, Smp. 166-168°C.

(±)-Phenyl-6-fluor-2,3-dihydro-2',4'-dioxospiro[4H-1-benzopyran-4,5'-thiazolidin]-8-carbamat, Smp. 198-200°C.

(±)-Benzyl-6-fluor-2,3-dihydro-2',4'-dioxospiro[4H-1-benzopyran-4,5'-thiazolidin]-8-carbamat, Smp. 214-216°C.

(±)-p-Nitrophenyl-6-fluor-2,3-dihydro-2',4'-dioxospiro-[4H-1-benzopyran-4,5'-thiazolidin]-8-carbamat, Smp. 160-162°C.

(±)-p-Nitrobenzyl-6-fluor-2,3-dihydro-2',4'-dioxospiro-[4H-1-benzopyran-4,5'-thiazolidin]-8-carbamat, Smp. 175-177°C.

(±)-p-(Methoxycarbonyl)phenyl-6-fluor-2,3-dihydro-2',4'-dioxospiro[4H-1-benzopyran-4,5'-thiazolidin]-8-carbamat, Smp. 159-161°C.

## Beispiel 11

268 mg (±)-8-Amino-6-fluor-2,3-dihydrospiro[4H-1-benzopyran-4,5'-thiazolidin]-2',4'-dion wurden in 2 ml Tetrahydrofuran mit 100 µl Aethyl-isocyanat bei Raumtemperatur während 2 1/2 Stunden gerührt; anschliessend wurde während weiteren 2 Stunden auf 50°C erwärmt. Die flüchtigen Anteile wurden unter vermindertem Druck entfernt, der Rückstand wurde an einer Säule mit Kieselgel mittels Hexan/Essigester 1:2 chromatographisch gereinigt, mit Aktivkohle in Methanol entfärbt und aus Essigester/Hexan umkristallisiert. Man erhielt den (±)-1-Aethyl-3-[6-fluor-2,3-dihydro-2',4'-dioxospiro[4H-1-benzopyran-4,5'-thiazolidin]-8-yl]harnstoff in Form farbloser Kristalle vom Smp. 197-199°C.

## Beispiel 12

5,144 g (±)-8-Amino-6-fluor-2,3-dihydrospiro[4H-1-benzopyran-4,5'-thiazolidin]-2',4'-dion wurden in 50 ml 25%ige wässerige Salzsäure eingetragen und im Eisbad mit einer Lösung von 1,45 g Natriumnitrit in 6ml Wasser tropfenweise versetzt. Nach 60 Minuten Rühren bei 0°C wurde das überschüssige Nitrit durch Zugabe einer Spatelspitze Harnstoff zerstört.

Daneben wurde eine Lösung von 20,59 g Kaliumcyanid in 100 ml Wasser bei 0°C in kleinen Portionen mit 8,58 g Kupfer(I)-cyanid versetzt. Anschliessend wurden 35,6 g Natriumbicarbonat eingetragen und die Lösung mit Essigester überschichtet.

Die oben bereitete Lösung des Diazoniumsalzes wurde nun zu

dieser Kupfercyanidlösung gegossen und das Gemisch, aus dem unter heftigem Schäumen Stickstoff entwich, eine halbe Stunde bei Raumtemperatur nachgerührt. Anschliessend wurde durch vorsichtige Zugabe von 25%iger Salzsäure auf pH 5,5 angesäuert und das Produkt durch Ausschütteln mit drei Portionen Essigester ausgezogen. Die organischen Phasen wurden mit ges. Kochsalzlösung nachgewaschen und mit Natriumsulfat getrocknet. Nach Einengen des Lösungsmittels wurde der Rückstand an Kieselgel mit Chloroform/Essigester 4:1 chromatographisch gereinigt und anschliessend aus einem Gemisch von Essigester und n-Hexan umkristallisiert. Man erhielt (±)-8-Cyan-6-fluor-2,3-dihydrospiro[4H-1-benzopyran-4,5'-thiazolidin]-2',4'-dion, gelbliche Kristalle vom Smp. 230-232°C.

## Beispiel 13

268 mg (±)-8-Amino-6-fluor-2,3-dihydrospiro[4H-1-benzopyran-4,5'-thiazolidin]-2',4'-dion wurden in 3 ml 50%ige Tetrafluorborsäure eingetragen und im Eisbad mit einer Lösung von 76 mg Natriumnitrit in 0,3 ml Wasser tropfenweise versetzt. Das Reaktionsgemisch wurde anschliessend während 90 Minuten bei Raumtemperatur gerührt und danach überschüssiges Nitrit durch Zugabe von etwas Harnstoff zerstört. Die Lösung des Diazoniumsalzes wurde auf einmal in eine Lösung von 12,08 g Kupfer(II)-nitrat in 25 ml Wasser eingetragen und kräftig gerührt. Nach Zugabe von einer kleinen Spatelspitze Kupfer(I)-oxid setzte heftige Gasentwicklung ein. Das Gemisch wurde bei Raumtemperatur gerührt bis zur Beendigung des Schäumens (ca. 90 Minuten). Das Produkt wurde darauf mit 3 Portionen Aether extrahiert, die organischen Phasen wurden viermal mit Wasser gewaschen und mit Natriumsulfat getrocknet. Der nach Einengen des Lösungsmittels verbliebene Rückstand wurde mittels Chromatographie an präparativen Dickschichtplatten getrennt und das (±)-6-Fluor-8-hydroxy-2,3-dihydrospiro[4H-1-benzopyran-4,5'-thiazolidin]-2',4'-dion als nicht kristallisierbares, festes Material isoliert.

Beispiel 14

2,00 g (±)-6-Fluor-2,3-dihydrospiro[4H-1-benzopyran-4,5'-thiazolidin]-2',4'-dion und 3,116 g Brucin wurden in 60 ml Aethanol zum Kochen erhitzt, wobei nach ca. 10 Minuten spontane Kristallisation einsetzte. Das Reaktionsgemisch wurde nach 15 Minuten auf Raumtemperatur abkühlen gelassen. Die Kristalle wurden auf einer Nutsche abgetrennt und lieferten nach dem Trocknen 2,538 g Material vom Smp. 146-148°C, $[\alpha]_D^{25}$ = +38,5° (c = 1 in $CHCl_3$). Diese Kristalle wurden in 50 ml Essigester aufgenommen und mit 25 ml 1N-Salzsäure während 15 Minuten kräftig durchgerührt. Die Essigester-Phase wurde abgetrennt und mit weiteren 25 ml Salzsäure und mit 25 ml ges. Kochsalzlösung nachextrahiert. Nach Trocknen mit Natriumsulfat wurde eingeengt und der Rückstand über eine Säule mit Kieselgel chromatographisch gereinigt (Elution mit Hexan/Aether 1:1). Es wurden 948 mg farbloses, amorphes Material isoliert, das nicht zur Kristallisation gebracht werden konnte; $[\alpha]_D^{25}$ = +140,2° (c = 1 in $CH_3OH$), das $^1$H-NMR-Spektrum deckt sich mit dem des racemischen Materials. Die Mutterlauge des Brucinsalzes wurde eingeengt und ergab 3,005 g nichtkristallisierbaren Schaum, aus dem in der gleichen Weise wie für das kristalline Material die Säure freigesetzt wurde und aus dem 938 mg amorphes Material gewonnen wurden; $[\alpha]_D^{20}$ = -139,4° (c = 1 in $CH_3OH$).

Beispiel A

Eine Tablette kann die folgende Zusammensetzung aufweisen:

| | |
|---|---|
| Wirkstoff der Formel I | 1-10 Gew.-Teile |
| Natriumcitrat | 5 Gew.-Teile |
| Alginsäure | 2 Gew.-Teile |
| Polyvinylpyrrolidon | 2 Gew.-Teile |
| Magnesiumstearat | 1 Gew.-Teile |

Die Bestandteile werden gemischt und zu Tabletten gepresst, die 20-200 mg Wirkstoff enthalten.

Patentansprüche

1. Verbindungen der allgemeinen Formel

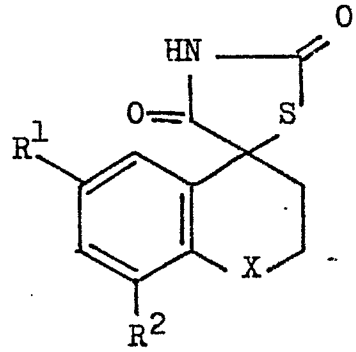

I

worin X Methylen, Sauerstoff oder Schwefel; $R^1$ Halogen; $R^2$ Wasserstoff, Nitro, Cyano, $OR^3$ oder $NHR^3$; oder beide Reste $R^1$ und $R^2$ gleichzeitig Wasserstoff oder Nitro; $R^3$ Wasserstoff, Alkyl, Acyl oder eine Gruppe $-CO-OR^4$ oder $-CO-NHR^4$; und $R^4$ Alkyl, Aryl oder Aralkyl bedeuten,

und deren Salze mit physiologisch verträglichen Kationen.

2. Verbindungen nach Anspruch 1, in denen X Methylen, Sauerstoff oder Schwefel; $R^1$ Halogen; $R^2$ Wasserstoff, Nitro, Cyano oder $NHR^3$; und $R^3$ Wasserstoff, Alkyl oder Acyl bedeuten.

3. Verbindungen nach Anspruch 1 und 2, in denen X Sauerstoff ist.

4. Verbindungen nach Anspruch 1-3, in denen $R^1$ Halogen ist.

5. (±)-6-Fluor-8-acetamido-2,3- dihydrospiro[4H-1-benzopyran-4,5'-thiazolidin]-2',4'-dion.

6. (±)-Methyl-6-fluor-2,3-dihydro-2',4'-dioxospiro[4H-1-benzopyran-4,5'-thiazolidin]-8-carbamat.

7. (±)-6-Fluor-2,3-dihydrospiro[4H-1-benzpyran-4,5'-thiazolidin]-2',4'-dion, 3-(4-Fluor-phenoxy)propionitril,

(±)-6-Fluor-8-nitro-2,3-dihydrospiro[4H-1-benzopyran-4,5'-thiazolidin]-2',4'-dion, (±)-6-Fluor-8-amino-2,3-dihydrospiro[4H-1-benzopyran-4,5'-thiazolidin]-2',4'-dion, 6-Fluor-2,3-dihydrospiro[4H-1-benzopyran-4,5'-thiazolidin]-2',4'-dion, (±)-6-Fluor-8-undecylamido-2,3-dihydrospiro-[4H-1-benzopyran-4,5'-thiazolidin]-2',4'-dion.

8. (±)-6-Fluor-8-hydroxy-2,3-dihydrospiro[4H-1-benzopyran-4,5'-thiazolidin]-2',4'-dion,

(±)-1-Aethyl-3-[6-fluor-2,3-dihydro-2',4'-dioxospiro-[4H-1-benzopyran-4,5'-thiazolidin]-8-yl]harnstoff,

(±)-8-Cyan-6-fluor-2,3-dihydrospiro[4H-1-benzopyran-4,5'-thiazolidin]-2',4'-dion,

(±)-Aethyl-6-fluor-2,3-dihydro-2',4'-dioxospiro[4H-1-benzopyran-4,5'-thiazolidin]-8-carbamat,

(±)-6-Chlor-2,3-dihydrospiro[4H-1-benzopyran-4,5'-thiazolidin]-2',4'-dion,

(±)-N-[6-Fluor-2,3-dihydro-2',4'-dioxospiro[4H-1-benzopyran-4,5'-thiazolidin]-8-yl]formamid,

(±)-Butyl-6-fluor-2,3-dihydro-2',4'-dioxospiro[4H-1-benzopyran-4,5'-thiazolidin]-8-carbamat,

(±)-Isobutyl-6-fluor-2,3-dihydro-2',4'-dioxospiro[4H-1-benzopyran-4,5'-thiazolidin]-8-carbamat,

(±)-Phenyl-6-fluor-2,3-dihydro-2',4'-dioxospiro[4H-1-benzopyran-4,5'-thiazolidin]-8-carbamat,

(±)-Benzyl-6-fluor-2,3-dihydro-2',4'-dioxospiro[4H-1-benzopyran-4,5'-thiazolidin]-8-carbamat,

(±)-p-Nitrophenyl-6-fluor-2,3-dihydro-2',4'-dioxospiro-[4H-1-benzopyran-4,5'-thiazolidin]-8-carbamat,

(±)-p-Nitrobenzyl-6-fluor-2,3-dihydro-2',4'-dioxospiro-[4H-1-benzopyran-4,5'-thiazolidin]-8-carbamat,

(±)-p-(Methoxycarbonyl)phenyl-6-fluor-2,3-dihydro-2',4'-dioxospiro[4H-1-benzopyran-4,5'-thiazolidin]-8-carbamat,

(±)-N-[6-Fluor-2,3-dihydro-2',4'-dioxospiro[4H-1-benzopyran-4,5'-thiazolidin]-8-yl]pivalamid,

(±)-N-[6-Fluor-2,3-dihydro-2',4'-dioxospiro[4H-1-benzopyran-4,5'-thiazolidin]-8-yl]-3,3-dimethylbutyramid,

(±)-N-[6-Fluor-2,3-dihydro-2',4'-dioxospiro[4H-1-benzopyran-4,5'-thiazolidin]-8-yl]benzamid,

(±)-p-Brom-N-2,3-dihydro-6-fluor-2',4'-dioxospiro[4H-1-benzopyran-4,5'-thiazolidin-8'-yl]benzamid,

(±)-N-[6-Fluor-2,3-dihydro-2',4'-dioxospiro[4H-1-benzopyran-4,5'-thiazolidin]-8-yl]-p-nitrobenzamid,

(±)-N-[6-Fluor-2,3-dihydro-2',4'-dioxospiro[4H-1-benzopyran-4,5'-thiazolidin]-8-yl]-3,5-dinitrobenzamid und

(±)-[[6-Fluor-2,3-dihydro-2',4'-dioxospiro[4H-1-benzopyran-4,5'-thiazolidin]-8-yl]carbamoyl]methylacetat.

9. Verbindungen nach Anspruch 1-8 zur Anwendung als Heilmittel.

10. Verbindungen nach Anspruch 1-8 zur Anwendung bei der Behandlung von Stoffwechselerkrankungen, insbesondere Diabetes mellitus.

11. Verfahren zur Herstellung von Verbindungen der allgemeinen Formel I und deren Salzen mit physiologisch verträglichen Kationen, dadurch gekennzeichnet, dass man eine Verbindung der allgemeinen Formel

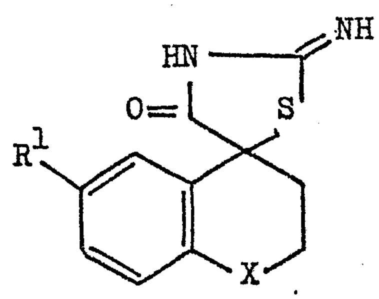

II

worin $R^1$ und X die in Anspruch 1 angegebene Bedeutung haben,

mit einer Säure behandelt, gewünschtenfalls das Reaktionsprodukt nitriert, gewünschtenfalls in einer so erhaltenen

Verbindung der Formel I, in der $R^1$ Halogen ist, die Nitrogruppe zur Aminogruppe reduziert und letztere gewünschtenfalls acyliert oder alkyliert oder durch die Cyano- oder Hydroxygruppe ersetzt; und letztere gewünschtenfalls alkyliert oder acyliert; und gegebenenfalls das Reaktionsprodukt in ein Salz mit einem physiologisch verträglichen Kation überführt.

12. Pharmazeutische Präparate, enthaltend eine Verbindung nach Anspruch 1 und einen pharmazeutischen Trägerstoff.

13. Verbindungen der Formel

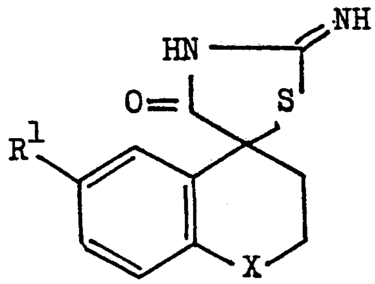

II

worin $R^1$ und X die in Anspruch 1 angegebene Bedeutung haben.

***

<u>Patentansprüche für Oesterreich</u>

1. Verfahren zur Herstellung von Verbindungen der allgemeinen Formel

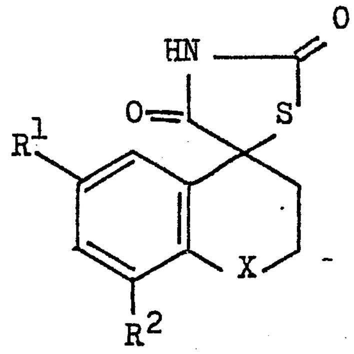

I

worin X Methylen, Sauerstoff oder Schwefel; $R^1$ Halogen; $R^2$ Wasserstoff, Nitro, Cyano, $OR^3$ oder $NHR^3$; oder beide Reste $R^1$ und $R^2$ gleichzeitig Wasserstoff oder Nitro; $R^3$ Wasserstoff, Alkyl, Acyl oder eine Gruppe $-CO-OR^4$ oder $-CO-NHR^4$; und $R^4$ Alkyl, Aryl oder Aralkyl bedeuten,

und deren Salze mit physiologisch verträglichen Kationen, dadurch gekennzeichnet, dass man eine Verbindung der allgemeinen Formel

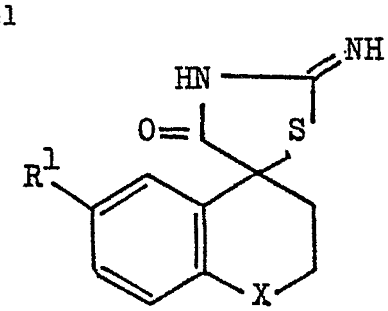

II

worin $R^1$ und X die in Anspruch 1 angegebene Bedeutung haben,

mit einer Säure behandelt, gewünschtenfalls das Reaktionsprodukt nitriert, gewünschtenfalls in einer so erhaltenen Verbindung der Formel I, in der $R^1$ Halogen ist, die Nitrogruppe zur Aminogruppe reduziert und letztere gewünschtenfalls acyliert oder alkyliert oder durch die Cyano- oder Hydroxygruppe ersetzt und letztere gewünschtenfalls alkyliert oder acyliert; und gegebenenfalls das Reaktionsprodukt in ein Salz mit einem physiologisch verträglichen Kation überführt.

2. Verfahren nach Anspruch 1 zur Herstellung von Verbindungen der Formel I in denen X Methylen, Sauerstoff oder Schwefel; $R^1$ Halogen; $R^2$ Wasserstoff, Nitro, Cyano oder $NHR^3$; und $R^3$ Wasserstoff, Alkyl oder Acyl bedeuten.

3. Verfahren nach Anspruch 1 zur Herstellung von Verbindungen von Anspruch 1, in denen X Sauerstoff ist.

4. Verfahren nach Anspruch 1 zur Herstellung von Verbindungen von Anspruch 1, in denen $R^1$ Halogen ist.

5. Verfahren nach Anspruch 1 zur Herstellung von (±)-6-Fluor-8-acetamido-2,3- dihydrospiro[4H-1-benzopyran-4,5'-thiazolidin]-2',4'-dion.

6. Verfahren nach Anspruch 1 zur Herstellung von (±)-Methyl-6-fluor-2,3-dihydro-2',4'-dioxospiro[4H-1-benzopyran-4,5'-thiazolidin]-8-carbamat.

7. Verfahren nach Anspruch 1 zur Herstellung von (±)-6-Fluor-2,3-dihydrospiro[4H-1-benzpyran-4,5'-thiazolidin]-2',4'-dion, 3-(4-Fluor-phenoxy)propionitril, (±)-6-Fluor-8-nitro-2,3-dihydrospiro[4H-1-benzopyran-4,5'-thiazolidin]-2',4'-dion, (±)-6-Fluor-8-amino-2,3-dihydrospiro[4H-1-benzopyran-4,5'-thiazolidin]-2',4'-dion, 6-Fluor-2,3-dihydrospiro[4H-1-benzopyran-4,5'-thiazolidin]-2',4'-dion, (±)-6-Fluor-8-undecylamido-2,3-dihydrospiro-[4H-1-benzopyran-4,5'-thiazolidin]-2',4'-dion.

8. Verfahren nach Anspruch 1 zur Herstellung von (±)-6-Fluor-8-hydroxy-2,3-dihydrospiro[4H-1-benzopyran-4,5'-thiazolidin]-2',4'-dion, (±)-1-Aethyl-3-[6-fluor-2,3-dihydro-2',4'-dioxospiro[4H-1-benzopyran-4,5'-thiazolidin]-8-yl]harnstoff, (±)-8-Cyan-6-fluor-2,3-dihydrospiro[4H-1-benzopyran-4,5'-thiazolidin]-2',4'-dion, (±)-Aethyl-6-fluor-2,3-dihydro-2',4'-dioxospiro[4H-1-benzopyran-4,5'-thiazolidin]-8-carbamat, (±)-6-Chlor-2,3-dihydrospiro[4H-1-benzopyran-4,5'-thiazolidin]-2',4'-dion, (±)-N-[6-Fluor-2,3-dihydro--2',4'-dioxospiro[4H-1-benzopyran-4,5'-thiazolidin]-8-yl]formamid, (±)-Butyl-6-fluor-2,3-dihydro-2',4'-dioxospiro[4H-1-benzopyran-4,5'-thiazolidin]-8-carbamat, (±)-Isobutyl-6-fluor-2,3-dihydro-2',4'-dioxospiro[4H-1-benzopyran-4,5'-thiazolidin]-8-carbamat, (±)-Phenyl-6-fluor-2,3-dihydro-2',4'-dioxospiro-[4H-1-benzopyran-4,5'-thiazolidin]-8-carbamat, (±)-Benzyl-6-fluor-2,3-dihydro-2',4'-dioxospiro[4H-1-benzopyran-4,5'-thiazolidin]-8-carbamat, (±)-p-Nitrophenyl-6-fluor-2,3-dihydro-2',4'-dioxospiro[4H-1-benzopyran-4,5'-thiazolidin]-8-carbamat, (±)-p-Nitrobenzyl-6-fluor-2,3-dihydro-2',4'-dioxospiro[4H-1-benzopyran-4,5'-thiazolidin]-8-carbamat, (±)-p-(Methoxycarbonyl)phenyl-6-fluor-2,3-dihydro-2',4'-dioxospiro[4H-1-benzopyran-4,5'-thiazolidin]-8-carbamat, (±)-N-[6-Fluor-2,3-dihydro-2',4'-dioxospiro[4H-1-benzopyran-4,5'-thiazolidin]-8-yl]pivalamid, (±)-N-[6-Fluor-2,3-dihydro-2',4'-dioxospiro[4H-1-benzopyran-4,5'-thiazolidin]-8-yl]-3,3-dimethylbutyramid, (±)-N-[6-Fluor-2,3-dihydro-2',4'-dioxospiro[4H-1-benzopyran-4,5'-thiazolidin]-8-yl]benzamid, (±)-p-Brom-N-2,3-dihydro-6-fluor-2',4'-dioxospiro[4H-1-benzopyran-4,5'-thiazolidin-8'-yl]benzamid, (±)-N-[6-Fluor-2,3-dihydro-2',4'-dioxospiro[4H-1-benzopyran-4,5'-thiazolidin]-8-yl]-p-nitrobenzamid, (±)-N-[6-Fluor-2,3-dihydro-2',4'-dioxospiro[4H-1-benzopyran-4,5'-thiazolidin]-8-yl]-3,5-dinitrobenzamid und (±)-[[6-Fluor-2,3-dihydro-2',4'-dioxospiro[4H-1-benzopyran-4,5'-thiazolidin]-8-yl]carbamoyl]methylacetat.